# EUROPÄISCHE PATENTSCHRIFT

(11) **EP 1 451 750 B1**
(45) Veröffentlichungstag und Bekanntmachung des Hinweises auf die Patenterteilung: **15.11.2006**
(21) Anmeldenummer: 02774776.5
(22) Anmeldetag: 11.11.2002
(51) Int. Cl.: G06F 19/00

(54) **VERFAHREN ZUR IDENTIFIKATION VON PHARMAKOPHOREN**
METHOD FOR THE IDENTIFICATION OF PHARMACOPHORES
PROCEDE POUR IDENTIFIER DES PHARMACOPHORES

(30) Priorität: 15.11.2001 DE 10156245
(43) Veröffentlichungstag der Anmeldung: 01.09.2004
(73) Patentinhaber: Bayer Technology Services GmbH, 51368 Leverkusen (DE)
(72) Erfinder: SCHUPPERT, Andreas, 51515 Kürten (DE)
(86) Internationale Anmeldenummer: PCT/EP2002/012549
(87) Internationale Veröffentlichungsnummer: WO 2003/042702

(56) Entgegenhaltungen:
- WO-A-00/49539
- WO-A-98/20437

## Beschreibung

Die vorliegende Erfindung betrifft ein Verfahren zur Identifikation eines molekularen Pharmakophors sowie ein entsprechendes Computerprogramm und Computersystem.

Die Suche nach molekularen Pharmakophoren aus experimentellen Daten ist ein entscheidender Schritt bei der Suche nach neuen Wirkstoffen. Aus dem Stand der Technik ist an sich bekannt für die Gewinnung der experimentellen Daten, die Reaktionen einer großen Zahl von definierten Substanzen aus einer Substanzbibliothek mit einem vorab definiertem Zielmolekül - dem sogenannten Target - zu untersuchen. Entsprechend der Reaktion mit dem Target werden die Substanzen der Substanzbibliothek klassifiziert. Eine Möglichkeit der Klassifizierung ist dabei eine binäre Klassifizierung, das heißt, zum Beispiel nach logisch "0", das heißt, keine Reaktion, und logisch "1", das heißt, es tritt eine Reaktion ein.

Für die Entwicklung eines Wirkstoffs ist es entscheidend, aus der Klassifizierung der einzelnen Substanzen und deren bekannter chemischer Struktur pharmakologisch relevante Untereinheiten (Pharmakophore) zu identifizieren. Dies beinhaltet auch die Identifikation von sog. Leitstrukturen, bei denen es sich um chemisch wohldefinierte, zusammenhängende Untereinheiten eines Moleküls handelt. Als Pharmakophor und insbesondere als Leitstruktur wird eine molekulare Untereinheit bezeichnet, die für die Reaktionsfähigkeit mit dem Target relevant ist. Dabei ist es unerheblich, ob der Beitrag einer Untereinheit die Reaktion verstärkt oder inhibiert. Die Pharmakophore müssen nicht notwendigerweise eine kompakte molekulare Untereinheit bilden. Es ist durchaus möglich, dass räumlich getrennte molekulare Untereinheiten kooperativ zur Wirkung beitragen.

Die biologischen oder chemischen Deskriptoren bzw. molekularen Strukturen werden in einem Inputvektor kodiert. Das Wirkungsprofil ist eine a priori unbekannte Funktion, die von der molekularen Struktur abhängt. Daher wird diese Funktion im weiteren Struktur - Wirkungsbeziehung (SWB) genannt. Aus deren funktionaler Form kann der Pharmakophor hergeleitet werden, indem die Wirkungsbeiträge der Inputvariablen zu wenigen Wirkungseinheiten verknüpft werden, die gemeinsam die SWB ergeben. (vgl. J. Bajorath, "Selected Concepts and Investigations in Compound Classification, Molecular Descriptor Analysis, and Virtual Screening", J. Chem. In Comput. Sci., 2001, 41, 233-2459.

Ist ein Pharmakophor identifiziert, dann kann durch systematische Variation derselben der Wirkstoff optimiert werden. Für die systematische Optimierung eines identifizierten Pharmakophors existieren etablierte Verfahren.

Für die Identifizierung von Pharmakophoren werden eine Kombination verschiedener Methoden eingesetzt:
1.) Definition von strukturellen Untergruppen der Molekülstrukturen (Fingerprints) sowie die Ermittlung von chemischen und/oder biologischen Deskriptoren zu den einzelnen Molekülstrukturen. Deskriptoren sind molekülspezifische chemische Größen (z.B. Acidität, Zahl der OH - Gruppen etc.) oder biologische Größen (wie Toxizität). Die Fingerprints werden in Form von binären Strings kodiert. Dabei bezeichnet jede Stelle des Strings eine molekulare Untergruppe. Es wird an einer Stelle des Strings eine 1 gesetzt, wenn die entsprechende Untergruppe in der Molekülstruktur vorhanden ist, ansonsten wird eine 0 gesetzt. Erfahrungsgemäß ist die Auswahl der molekularen Untergruppen für den Erfolg der Pharmakophoridentifizierung wichtig und Gegenstand aktueller Forschung (vgl. US Patent No. 6.240.374 und US Patent No. 6.208.942). Bei den Fingerprints können nicht nur das Vorhandensein von Untergruppen kodiert werden, sondern auch deren Zusammenhang in der chemischen Struktur des Moleküls. Die Entwicklung optimaler, generisch einsetzbarer, Fingerprints ist jedoch äquivalent zu Identifizierung von Pharmakophoren und noch nicht gelöst.
2.) Auf die Fingerprints werden Datenreduktionsverfahren angewandt. Die gebräuchlichsten hierzu sind Hauptkomponentenanalyse (PCA: Principal Component Analysis) und Clusterungsverfahren. Hierdurch werden die sehr langen Strings erheblich reduziert, wobei die Komplexität des Problems der Identifizierung von Pharmakophoren reduziert wird. Da alle hierfür existierenden Verfahren heuristisch sind und keinerlei Informationen über die Wirkungsstruktur enthalten, besteht die Gefahr, bei der Reduktion Informationen zu eliminieren, die für die Wirkung relevant sind. Methoden, dieses systematisch zu vermeiden, existieren nicht.
3.) Auf die (reduzierten) Datensätze werden etablierte Verfahren des Data Mining angewandt, um Struktur - Wirkungsbeziehungen zwischen Fingerprints/Deskriptoren und der pharmakologischen Wirkung zu finden.

Die gebräuchlichsten Verfahren sind
- Entscheidungsbäume,
- Assoziationsregeln,
- Neuronale Netze.

Bei Entscheidungsbäumen und Assoziationsregeln wird mit verschiedenen kombinatorischen Methoden versucht, eine Beschreibung der Struktur-Wirkungsbeziehung unter Verwendung möglichst weniger Variablen zu erzielen. Ein solches Verfahren kann daher für die Wirkung relevante und nicht relevante Strukturvariablen voneinander trennen. Nachteilig ist, dass dabei prinzipiell nur solche Wirkungseinheiten als relevant identifiziert werden können, die einen positiven oder negativen Beitrag zur Wirkung unabhängig von der Belegung der übrigen Strukturvariablen haben. In dem häufigen Fall, dass eine Wechselwirkung zwischen mehreren Wirkungseinheiten auftritt, kann sie nur dann identifiziert werden, wenn stets eine Verstärkung oder Abschwächung der Gesamtwirkung auftritt.

In allen Fällen, in denen aus strukturchemischen Gründen eine komplexe Wechselwirkung zwischen Wirkungseinheiten auftritt, kann diese von den oben genannten Verfahren nicht identifiziert werden. In diesen Fällen werden auch die Gruppierungen der Strukturvariablen zu Wirkungseinheiten nicht erkannt. Ein weiterer Nachteil der Verfahren ist, dass komplexe, mehrstufige Interaktionen zwischen Wirkungseinheiten grundsätzlich nicht erkannt werden können.

Im Gegensatz zu Entscheidungsbäumen und Assoziationsregeln lernen Neuronale Netze die SWB anhand der vorliegenden Daten "auswendig". Sie sind in der Lage, auch komplexe Interaktionen von vielen Variablen richtig abzubilden. Ihr entscheidender Nachteil ist, dass sie nur eine formale SWB liefern können. Explizite Informationen über eine funktionale Strukturierung der SWB können nicht gewonnen werden. Damit ist ihr Beitrag für die Identifizierung von Pharmakophoren darauf beschränkt, dass sie eine kompakte Darstellung der SWB sowie Interpolationen zwischen gemessenen Variablenbelegungen ermöglichen. Für die Strukturierung der SWB können sie konstruktionsbedingt keinen direkten Beitrag leisten. Eine chemisch relevante Identifizierung eines Pharmakophor ist daher nur sehr bedingt möglich. Ein zweiter Nachteil besteht darin, dass die hohe Flexibilität der neuronalen Netze dazu führt, dass bei den vorliegenden hochdimensionalen Datensätzen die Zuverlässigkeit des Vorhersagen durch ein neuronales Netz durch Overfitting stark abnimmt.

Verfahren, welche die explizite Integration von Vorwissen erlauben und zusätzlich aus den Daten Informationen über die funktionale Struktur der SWB generieren, sind nicht bekannt.

Auf der anderen Seite konnte in neuester Zeit die explizite Integration von Vorwissen in neuronale Netzstrukturen in Form strukturierter Hybridmodelle demonstriert und die dadurch erlangte Effizienzsteigerung bei der Modellierung komplexer Zusammenhänge nachgewiesen werden (vgl. A. Schuppert, Extrapolability of Structured Hybrid Models: a Key to Optimization of Complex Processes, in: Proceedings of EquaDiff 99, Fiedler, Gröger, Sprekels Eds., World Scientific Publishing, 2000).

Strukturierte Hybridmodelle enthalten neuronale Netze, die miteinander entsprechend der a priori vorgegebenen funktionalen Struktur der SWB verschaltet sind. Die als neuronale Netze realisierten Wirkungseinheiten werden dann ähnlich wie unstrukturierte neuronale Netze anhand der vorliegenden Daten trainiert. Es konnte gezeigt werden, dass hierdurch das Problem des Overfittings stark reduziert werden kann. Zusätzlich ermöglichen strukturierte Hybridmodelle eine Extrapolation der Daten, die mit reinen neuronalen Netzen prinzipiell unmöglich ist.

Für die Anwendung in der Pharmakophoridentifizierung ist die strukturierte Hybridmodellierung nicht anwendbar, solange die funktionale Struktur der gesuchten SWB nicht a priori bekannt ist. Da dies im Allgemeinen nicht der Fall ist, entfällt eine entscheidende Voraussetzung für den Einsatz von strukturierten Hybridmodellen. Im Gegenteil, die Aufklärung der funktionalen Struktur der SWB ist selbst die entscheidende Komponente bei der Pharmakophorsuche.

Es ist bisher jedoch nicht gelungen, in umgekehrter Weise aus vorliegenden Daten die funktionale Struktur der SWB zu bestimmen. Im Stand der Technik fehlt es also an sicheren Methoden für die Identifizierung von Pharmakophoren für ein gegebenes Target.

Patent WO 98/20437 (3-DIMENSIONAL PHARMACEUTICALS, INC.) offenbart ein Struktur-Eigenschaft-Modellgenerator, der für die Erstellung von Struktur-Eigenschaft-Modellen zur automatischen Identifikation und Erstellung chemischer Wirkstoffe mit gewünschten Eigenschaften verantwortlich ist. Als Ausführungsbeispiele von Struktur-Eigenschaft-Modellen werden strukturierte Hybridmodelle gegeben, die neuronale Netze beinhalten. Es werden jedoch keine Algorithmen zur Strukturierung dieser Modelle dargestellt.

Der Erfindung liegt daher die Aufgabe zu Grunde ein Verfahren zur Identifikation von molekularen Pharmakophoren sowie ein entsprechendes Computerprogramm und Computersystem zu schaffen.

Die der Erfindung zu Grunde liegende Aufgabe wird mit den Merkmalen der unabhängigen Patentansprüche jeweils gelöst. Bevorzugte Ausführungsformen der Erfindung sind in den abhängigen Patentansprüchen angegeben.

Ein vorteilhaftes Anwendungsgebiet der vorliegenden Erfindung ist die Identifikation von molekularen Pharmakophoren für die Zwecke der pharmakologischen Wirkungs-analyse. Insbesondere erlaubt es die Erfindung, die Entwicklung eines pharmakologischen Wirkstoffs wesentlich zu beschleunigen und dabei gleichzeitig die Kosten stark zu reduzieren.

Ein besonderer Vorteil der Erfindung liegt darin, dass sie die direkte Identifizierung der funktionalen Struktur der SWB aus gemessenen Struktur-Wirkungsdaten erlaubt.

Nach einer bevorzugten Ausführungsform der Erfindung wird vorausgesetzt, dass sich die Daten so klassifizieren lassen, dass die Wirkung eines jeden Datensatzes einer binären Darstellung zugänglich ist, das heißt, für die Zustände "nicht wirksam" und "wirksam". Nach einer weiteren bevorzugten Ausführungsform der Erfindung wird ferner vorausgesetzt, dass jede Wirkungseinheit des Pharmakophors ebenfalls nur zwei Zustände annehmen kann, nämlich "aktiv" und "inaktiv". Eine Wirkungseinheit wird dabei als "Blackbox" betrachtet.

Nach einer weiteren bevorzugten Ausführungsform der Erfindung werden die Wirkungen in mehr als zwei Klassen eingeteilt und codiert. Im Vergleich zu der binären Codierung erlaubt diese Ausführungsform neben der Unterscheidung "nicht wirksam" und "wirksam", unterschiedliche Abstufungen der Wirksamkeit mit in die Auswertung einzubeziehen. Entsprechend ist es auch möglich für jede Wirkungseinheit mehr als zwei Zustände zuzulassen.

Der Erfindung liegt die Erkenntnis zu Grunde, dass es eine Eigenschaft von strukturierten Hybridmodellen ist, dass zu jeder funktionalen Struktur der SWB ein genau definiertes System von invarianten Mengen in den Daten gehört. Das erfindungsgemäße Verfahren beruht darauf, dass die (eventuell vorhandenen) invarianten Mengen aus den Daten herausgefiltert werden, um daraus die SWB zu rekonstruieren. (Strukturierte Hybridmodelle sind an sich aus A. Schuppert, Extrapolability of Structured Hybrid Models: a Key to Optimization of Complex Processes, in: Proceedings of EquaDiff 99, Fiedler, Gröger, Sprekels Eds., World Scientific Publishing, 2000 bekannt.)

Für den Fall, dass eine Wirkungseinheit nur zwei Zustände annehmen kann, nämlich "aktiv" und "inaktiv", muss daher eine Clusterung der Belegungen der Eingangsvariablen jeder Wirkungseinheit gegeben sein, so dass unter allen Umständen für alle Belegungen einer der betreffenden Variablen der Ausgang der Wirkungseinheit logisch "0" ist und für alle Belegungen der anderen Variablen stets "1". Diese erzwungene Clusterung der Belegungen der Eingangsvariablen führt direkt zur Existenz von invarianten Mengen in der SWB.

Ein besonderer Vorteil der Erfindung liegt darin, dass aus einem vorgegebenen System von invarianten Mengen der SWB die funktionale Struktur der SWB rekonstruiert werden kann, insbesondere wenn die SWB eine Baumstruktur besitzt. Das erfindungsgemäße Verfahren setzt für die Berechnung der funktionalen Struktur der SWB weder die explizite Berechnung der genauen Belegung der Eingangs- und Ausgangsbeziehungen der einzelnen Wirkungseinheiten noch eine kombinatorische Variation aller möglichen funktionalen Strukturen voraus. Aufgrund dessen ist das erfindungsgemäße Verfahren besonders effizient und erlaubt es auch komplexe Probleme mit relativ geringem Berechnungsaufwand zu lösen.

Im Weiteren werden bevorzugte Ausführungsbeispiele der Erfindung mit Bezugnahme auf die Zeichnungen näher erläutert. Es zeigen
- Figur 1: eine Prinzipdarstellung für die Identifikation einer pharmakologischen Struktur-Wirkungsbeziehung,
- Figur 2: ein Beispiel für den formalen Aufbau eines Pharmakophors,
- Figur 3: ein Beispiel für ein strukturiertes Hybridmodell,
- Figur 4: ein Beispiel für eine Struktur-Wirkungsbeziehung bestehend aus Wirkungseinheiten mit jeweils binärem Eingang-Ausgangsverhalten,
- Figur 5: ein Flussdiagramm für die Berechnung verschiedener Variationen von Deskriptoren.
- Figur 6: ein Flussdiagramm für die Identifizierung von Wirkungseinheiten,
- Figur 7: ein Flussdiagramm eines Verfahrens für die experimentelle Ermittlung von Substanzen einer Substanzbibliothek auf ein Zielmolekül,
- Figur 8: eine Tabelle mit Deskriptoren der Substanzen der Substanzbibliothek und den experimentelle ermittelten Reaktionen,
- Figur 9: ein Flussdiagramm für eine Ausführungsform der Ermittlung der binären Variationen,
- Figur 10: eine Tabelle für die Bestimmung der binären Variationen gemäß der Figur 9,
- Figur 11: ein Flussdiagramm für die Bestimmung von ternären Variationen,
- Figur 12: eine Tabelle mit Variablenpaar-Kandidaten für die Zuordnung zu einer gemeinsamen Wirkeinheit und eine Tabelle mit Mengen von Variablen für die Variablenpaar-Kandidaten mit konfliktfreien Clustern.
- Figur 13: ein weiteres Beispiel für eine Struktur-Wirkungsbeziehung,

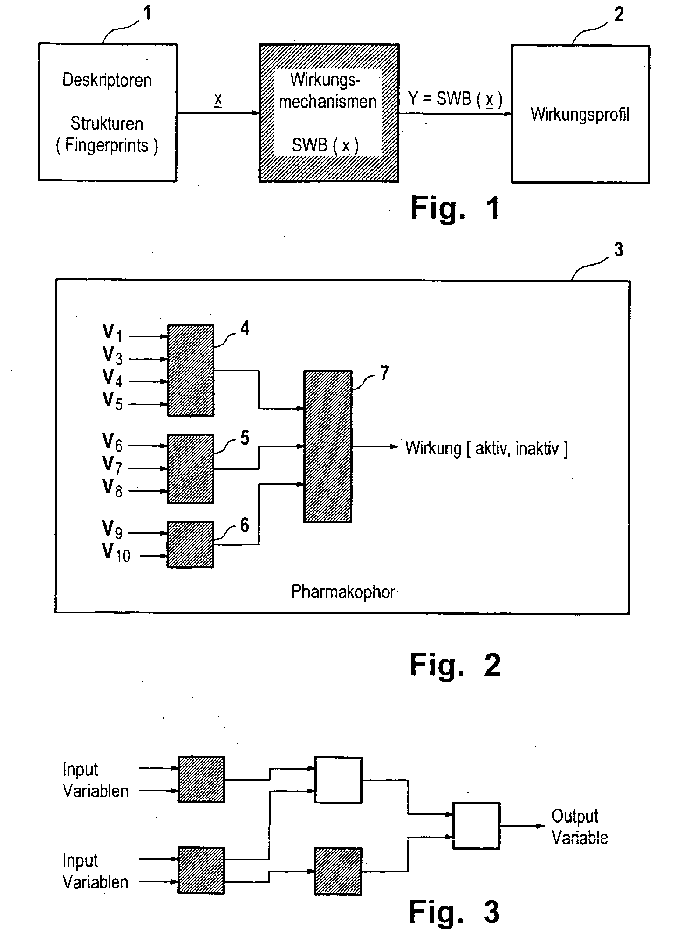

Die Figur 1 veranschaulicht das der Erfindung zu Grunde liegende Identifikationsproblem, insbesondere für pharmakologische Anwendungen. Eine Datenbank 1 beinhaltet die Deskriptoren der Substanzen einer Substanzbibliothek. Die Deskriptoren sind dabei binär codiert und beschreiben die Strukturen der Substanzen. Solche Deskriptoren werden auch als "Fingerprints" bezeichnet. Solche "Fingerprints" sind an sich aus dem Stand der Technik bekannt (vgl. J. Bajorath, Selected Concepts and Investigations in Compound Classification, Molecular Descriptor Analysis, and Virtual Screening, J. Chem. In. Comput. Sci., 2001, 41, 233-245).

Die Deskriptoren der Datenbank 1 stehen als Vektoren x am Ausgang der Datenbank 1 zur Verfügung und werden über den zu ermittelnden Wirkungsmechanismus der Struktur-Wirkungsbeziehung SWB (x) auf ein Wirkungsprofil abgebildet. Bei dem Wirkungsprofil handelt es sich um experimentell ermittelte Daten, die in einer Datenbank 2 abgespeichert sind. Zur Bestimmung des Wirkungsprofils wird möglichst für jeden einzelnen Deskriptor mittels eines Experiments ermittelt, ob die betreffende Substanz mit dem Zielmolekül, dem sogenannten Target, reagiert oder nicht.

Durch das Zielmolekül wird also eine Abbildung Y=SWB (x) von Substanzen, die mittels der Deskriptoren beschrieben werden, auf ein Wirkungsprofil geleistet. Das Identifikationsproblem besteht nun darin, aus den Eingangs- und Ausgangsgrößen der SWB, das heißt aus den Deskriptoren und dem Wirkungsprofil, auf die Struktur der SWB rückzuschließen.

Eine SWB ist als sogenanntes Pharmakophor gemäß Figur 2 darstellbar. Ein Pharmakophor kann eine oder mehrere Leitstrukturen umfassen.

Die Figur 2 zeigt einen Pharmakophor 3 mit den Wirkungseinheiten 4, 5, 6 und 7. Die Wirkungseinheit 4 hat als Eingänge die Variablen V₁, V₃, V₄ und V₅. Die Wirkungseinheit 5 hat als Eingänge die Variablen V₆, V₇ und V₈. Die Wirkungseinheit 6 hat die Eingänge V₉ und V₁₀. Die Wirkungseinheiten 4, 5 und 6 haben jeweils einen Ausgang der mit einem Eingang der Wirkungseinheit 7 verknüpft ist. Der Ausgang der Wirkungseinheit 7 gibt dann die Gesamtwirkung, das heißt, "aktiv" oder "inaktiv" an.

Die Figur 3 zeigt ein Beispiel für die typische Strukturierung von "strukturierten Hybridmodellen". Der funktionale Zusammenhang zwischen den Inputvariablen und den Outputvariablen wird durch den Zusammenhangsgrafen der Figur 3 repräsentiert. Durch die schwarzen Rechtecke werden dabei quantitativ unbekannte Funktionen repräsentiert, wogegen die weißen Rechtecke quantitativ bekannte Abhängigkeiten darstellen. Um die Vorteile einer strukturierten Hybridmodellierung nutzen zu können, ist es nicht notwendig, dass überhaupt bekannte Abhängigkeiten (weiße Rechtecke) im Modell enthalten sind. Diese Erkenntnis macht sich die Erfindung für das automatische Auffinden einer SWB aus Deskriptoren und einem mit Hinblick auf ein Target ermittelten Wirkungsprofil zu Nutze.

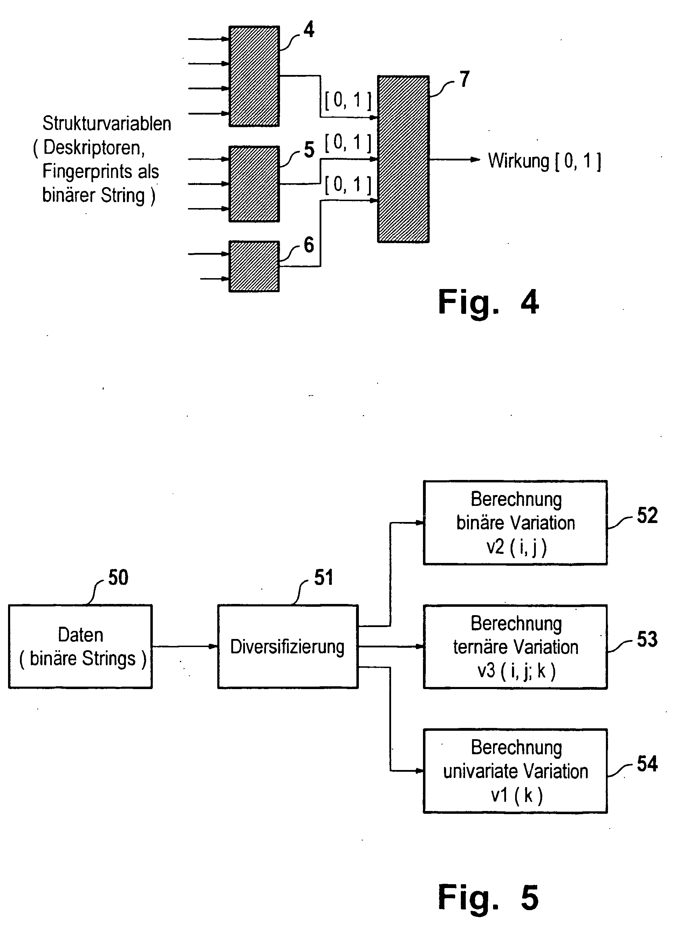

Die Figur 4 zeigt ein weiteres bevorzugtes Ausführungsbeispiel der Erfindung, bei dem die einzelnen Wirkungseinheiten jeweils nur zwei Zustände, das heißt logisch "Null" und logisch "Eins", entsprechend "aktiv" oder "inaktiv", annehmen können.

Die Figur 5 zeigt ein Flussdiagramm einer Ausführungsform des erfindungsgemäßen Verfahrens. In dem Schritt 50 werden die Deskriptoren der Substanzen einer Substanzbibliothek, für die ein Wirkungsprofil ermittelt worden ist, bereit gestellt. Die Bereitstellung erfolgt in der Form einer Datei aus den binären Deskriptoren der entsprechenden Molekülstrukturen mit einer einheitlichen Länge n.

Zu jeder der Molekülstrukturen ist zuvor die Zuordnung zur Gruppe der aktiven oder inaktiven Moleküle bezüglich der zu untersuchenden Wirkung bestimmt worden; diese Zuordnungen liegen in der Form des Wirkungsprofils vor. Die in dem Schritt 50 bereit gestellten binären Deskriptoren werden in dem Schritt 51 diversifiziert, das heißt, der jeweiligen Wirkung zugeordnet. Diversifizierung bedeutet hier, dass für jeden möglichen binären String aus Deskriptoren der Länge n die zugehörige Wirkung bekannt sein muss.

Ist dies mit den gegebenen Daten nicht der Fall, muss in einem Datenvorverarbeitungsschritt die Diversifizierung künstlich durchgeführt werden, entweder durch Clusterung der Datensätze in einzelne Cluster mit geringerer Variation in den Molekülstrukturen oder durch Interpolation mit Hilfe eines neuronalen Netzes. Durch die Clusterung wird erreicht, dass in jedem Cluster alle Molekülstrukturen durch binäre Strings mit kürzerer Länge m < n beschrieben werden können. Innerhalb der einzelnen Cluster ist die Diversifizierung einfacher zu erreichen als für das Gesamtensemble. Eine zusätzliche Möglichkeit zum Erreichen der Diversifizierung ist die systematische Eliminierung korrelierter Substrings aus den binären Deskriptoren.

Nach der Diversifizierung in Schritt 51 werden in den Schritt 52, 53 und 54 binäre, ternäre bzw. univariate Variationen berechnet. Dies dient zur Berechnung eines vollständigen Systems von invarianten Gruppen in der Datenmenge. Hierbei werden alle Tupel aus Variablen Vᵢ, Vⱼ der binären Deskriptoren-Strings gebildet. Zu jedem Tupel Vᵢ, Vⱼ werden zwei Größen berechnet:
- die binäre Variation v2(i,j). Sie wird berechnet, indem
   a) für alle 4 Belegungen der Variablen (i,j) ((0,0),(0,1),(1,0),(1,1)) die Wirkung des Gesamtsystems für jeweils alle Kombinationen der übrigen Parameter gesucht wird.
   b) Dann werden die Korrelationen cor(k,1), k,l = 1...4 der Wirkungsstruktur zwischen den Belegungen von (i,j) so berechnet, dass eine Belegung (z.B. (0,0)) mit einer anderen Belegung (z.B. (0,1)) dann korreliert ist, wenn die Wirkungen des Gesamtsystems für beide Belegungen unter allen Variationen in den Restvariablen stets identisch sind. Bei fehlerbehafteten Datensätzen wird nicht die exakte Identität gefordert, sondern ein vorgegebene Wahrscheinlichkeit, dass die Wirkungen in den Variationen der Restvariablen identisch sind. Cor(k,1) wird genau dann gleich 1 gesetzt, wenn die Belegung k mit der Belegung 1 wie beschrieben korreliert ist, ansonsten wir cor(k,1) auf 0 gesetzt.
   c) Im nächsten Schritt werden mit Hilfe bekannter Verfahren die Belegungen so geclustert, dass in jedem Cluster nur untereinander korrelierte Belegungen enthalten sind.
   d) Die binäre Variation v2(i,j) ist die Zahl der ermittelten Cluster.
- die ternäre Variation v3(i,j;k), die nach folgendem Algorithmus berechnet wird:
   a) Es werden zu jeder der 4 Belegungen des Variablentupels (i,j) (i,j = 1,....,n), und jeder der beiden Belegungen der zusätzlichen Variablen k die Wirkungen für jeweils alle Variationen der restlichen Variablen gesucht.
   b) Für jedes Tupel (i,j) und alle Variationen der restlichen Variablen wird geprüft, wie sich die Wirkung bei Sprung der Belegung der Variablen k von 0 auf 1 ändert. In den Fällen, in denen die Wirkung von der Belegung der Variablen (i,j) abhängt, wird geprüft, ob für k = 0 und k = 1 dieselbe Gruppierung der Wirkung bezüglich der Belegungen von (i,j) vorliegt.
   c) Die ternäre Variation v3(i,j;k) ist die Anzahl aller Variationen der Restvariablen, bei denen sowohl für den Fall k = 0 als auch k = 1 die Wirkung von der Belegung der Variablen (i,j) abhängt und für k = 0 und k = 1 jeweils unterschiedliche Gruppierungen in den (i,j) - Belegungen bezüglich der Wirkung auftreten.
- zusätzlich wird die Variation v1(k) berechnet, welche die Anzahl der Variationen der Restvariablen angibt, bei denen sich die Wirkung ändert, wenn eine Variable k von 0 auf 1 gesetzt wird.

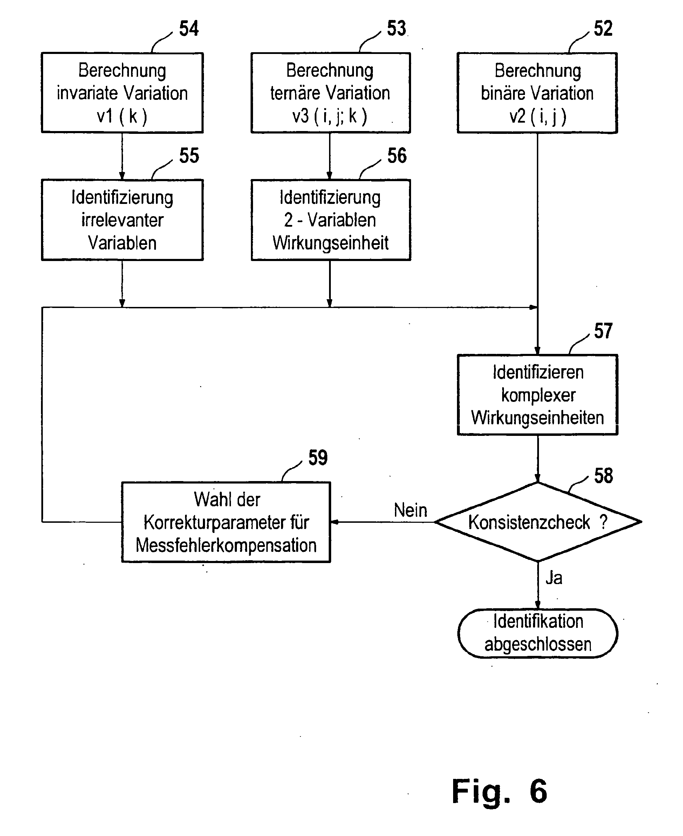

Die Figur 6 zeigt, wie von den Schritten 52, 53 bzw. 54 ausgehend weiter verfahren wird.

Mit Hilfe der binären und ternären Variationen v2(i,j) und v3(i,j;k) kann die funktionale Struktur der SWB eindeutig identifiziert werden. Hierzu werden zuerst die irrelevanten Variablen identifiziert (Schritt 55). Als irrelevante Variablen werden diejenigen Variablen bezeichnet, die keinerlei Einfluss auf die Wirkung zeigen. Diese lassen sich mit Hilfe von v1(k) sofort identifizieren:
- Eine Variable k heißt irrelevant, wenn v1(k) = 0 gilt.
   Alle irrelevanten Variablen werden aus dem Inputstring eliminiert. Danach (Schritt 56) werden diejenigen Variablentupel identifiziert, die schon als Tupel eine 2-Variablen Wirkungseinheit (2-WE) bilden:
   Ein Variablentupel (i,j), das keine irrelevante Komponente enthält, bildet eine 2-WE, wenn
- v2(i,j) = 2 gilt.
   Danach wird für alle Variablen, die nicht schon in einer 2-WE enthalten sind, überprüft, ob sie in einer komplexeren Wirkungseinheit enthalten sind (Schritt 57).
   Hierzu wird nach folgendem Algorithmus vorgegangen:
   a) Für alle (i,j) wird mit Hilfe der zugehörigen ternären Variationen v3(i,j;k), k = 1,...,n, die Menge Mk(i,j) derjenigen k - Variablen gesucht, für die v3(i,j;k) = 0 gilt.
   b) Dann werden alle Cluster aus (i,j) - Tupel gesucht, für die jedes zugehörige Clusterelement dieselbe Mk(i,j) - Menge hat.
   c) Alle Variablen, die in Tupeln vorkommen, die zu demselben Cluster gehören, bilden eine Wirkungseinheit.

Dieser Algorithmus erlaubt es, aus gemessenen Daten sowohl die irrelevanten Variablen zu identifizieren als auch die funktionale Struktur der SWB auf direktem Weg zu bestimmen.

Bei Daten, die ein Rauschen enthalten, d.h., bei denen die Wirkungszuordnung zu einer Molekülstruktur fehlerhaft sein kann, führt die folgende Modifikation des Algorithmus zum Ziel:

In Schritt 55 wird nicht mehr geprüft, ob v1 = 0, v2 = 2 und v3 = 0 ist, sondern es wird eine Fehlerbandbreite zugelassen. Das heißt, eine Variable gilt als irrelevant, wenn v1 kleiner als eine vorgegebene Grenze v1_crit ist. Die Kompensation von Fehlern bei der Identifizierung von 2-WE's wurde schon in der Beschreibung des Identifizierungsalgorithmus gezeigt. Bei der Identifizierung von komplexen Wirkungseinheiten wird die Fehlerkompensation so durchgeführt, dass in Schritt a) alle k-Variablen in Mk(i,j) eingestellt werden, für die v3(i,j;k) kleiner als eine vorgegebene Größe v3_crit ist.

Dieser Algorithmus ist ein direktes Verfahren, indem die funktionale Struktur der SWB direkt aus den Daten konstruiert wird. Sie hat im Gegensatz zu indirekten Verfahren, bei denen mögliche Strukturen auf Kompatibilität zu den Daten getestet werden, den Vorteil, dass die optimale Wahl der kritischen Parameter v1_crit, v2_crit und v3_crit dadurch unterstützt wird, dass das Ergebnis konsistent sein muss. Dies bedeutet:
- Alle Variablen müssen genau einer Wirkungseinheit zugewiesen sein oder als irrelevante Variable gekennzeichnet sein.
- Es darf keine Überschneidungen in der Zuordnung geben.

Alle Tests haben bisher gezeigt, dass bei einer Wahl der Variablen, die zu einer konsistenten Struktur führte, stets die richtige Struktur generiert wurde. Damit ist die Konsistenzprüfung ein scharfer Test für die Validierung der gefundenen funktionalen Struktur der SWB.

In dem Schritt 58 des Flussdiagramms der Figur 6 wird die Konsistenz der identifizierten Wirkungseinheiten geprüft. Wenn die Konsistenz nicht gegeben ist, wird die Wahl von Korrekturparametern für die Messfehler-Kompensation in dem Schritt 59 angepasst. Die Schritte 55 und/oder 56 und/oder 57 werden dann erneut ausgeführt und die entsprechenden Ergebnisse erneut einem Konsistenz-Check in dem Schritt 58 unterworfen. Wenn die Konsistenz gegeben ist, ist die Identifikation der Wirkungseinheiten damit abgeschlossen.

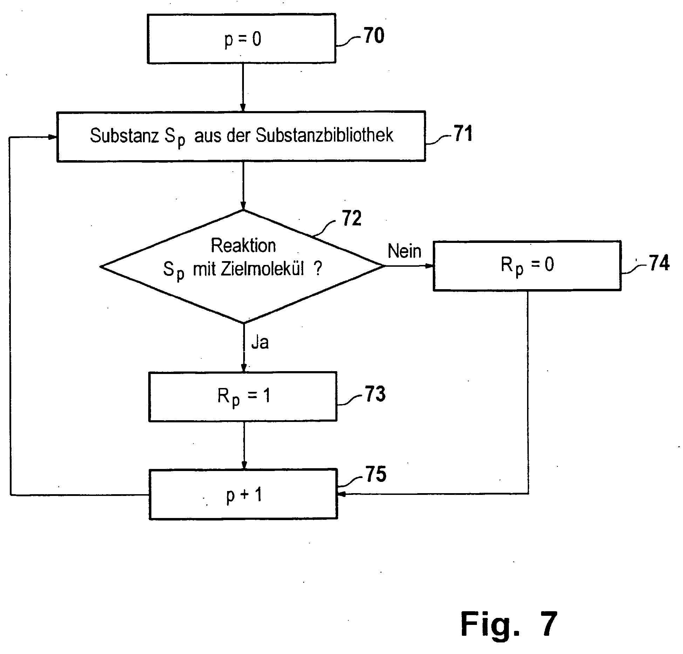

Anhand der Figuren 7 bis 11 wird im Weiteren ein bevorzugtes Ausführungsbeispiel des erfindungsgemäßen Verfahrens näher erläutert.

Die Figur 7 veranschaulicht zunächst die Vorgehensweise zur Erlangung der für die Durchführung dieses Verfahrens erforderlichen experimentellen Daten. Das Verfahren der Figur 7 kann weitgehend vollautomatisch von einem Laborautomaten durchgeführt werden.

In dem Schritt 70 wird zunächst der Index p initialisiert, das heißt, p= 0.

In dem Schritt 71 wird auf die Deskriptoren-Datenbank (vgl. Datenbank 1 der Figur 1) zugegriffen, um den Deskriptor für die Substanz Sₚ aus der Substanzbibliothek auszulesen. Insgesamt ist eine Menge von q Deskriptoren in der Datenbank vorhanden.

In dem Schritt 72 wird dann experimentell geprüft, ob die entsprechende Substanz Sp mit einem Zielmolekül reagiert, das heißt eine bestimmte Wirkung zeigt oder nicht. Wenn die Reaktion eintritt, wird in dem Schritt 73 das Datenfeld Rp für den Deskriptor der Substanz Sₚ gleich 1 gesetzt, im gegenteiligen Fall wird das Datenfeld Rp in dem Schritt 74 auf 0 gesetzt.

Danach wird in dem Schritt 75 der Wert des Index p inkrementiert. Die Schritte 71, 72 und 73 bzw. 74 werden dann erneut für den inkrementierten Index, das heißt für die nächste Substanz ausgeführt.

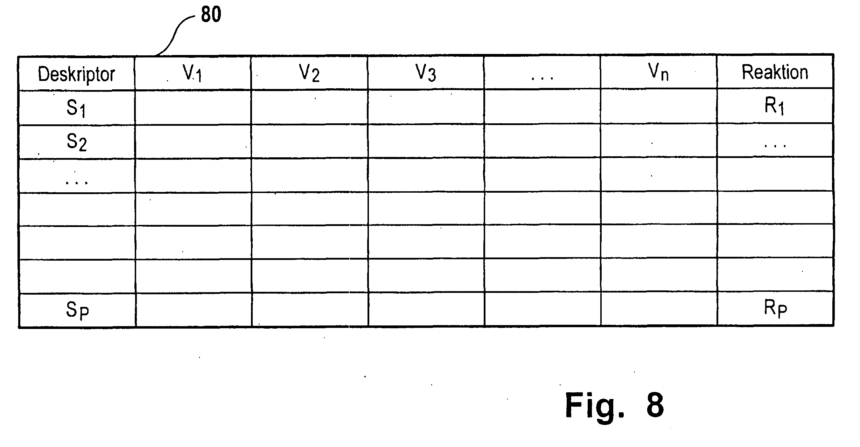

Die experimentell ermittelten Ergebnisse, das heißt, das Wirkungsprofil, wird in einer Tabelle 80 der Figur 8 zusammengefasst. Die Tabelle 80 beinhaltet für jede der Substanzen S₁, S₂, ..., Sₚ einen Deskriptor mit den Variablen V₁, V₂, V₃, ..., Vₙ. Ferner ist jedem dieser Deskriptoren ein Datenfeld Rp zugeordnet, welches in binär codierte Form angibt, ob im Experiment eine Reaktion stattgefunden hat oder nicht. Dem Deskriptor für die Substanz S₁ in der ersten Zeile der Tabelle 80 ist entsprechend das Datenfeld R₁ zugeordnet, welches entweder den Wert Null oder Eins aufweist, je nachdem, ob die Substanz S₁ im Experiment mit dem Target reagiert hat oder nicht. Die Tabelle 80 beinhaltet also die diversifizierten Daten (vgl. Schritt 51 der Figur 5).

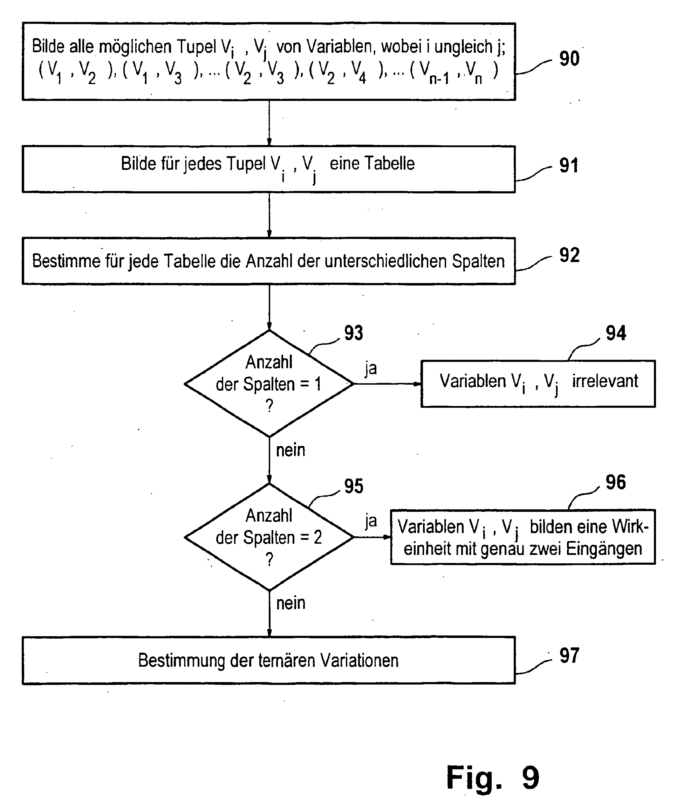

Die Figur 9 zeigt ein Flussdiagramm einer Ausführungsform eines Verfahrens zur Berechnung der binären Variationen (vgl. Schritt 52 der Figur 9).

In dem Schritt 90 werden zunächst alle möglichen Zwei-Tupel von Variablen Vᵢ und Vⱼ gebildet, wobei i ≠ j ist. Wenn binäre Deskriptoren verwendet werden, die jeweils eine Anzahl von n Variablen V₁, V₂, V₃,..., Vₙ aufweisen, werden also alle möglichen Paarungen unterschiedlicher Variablen Vᵢ und Vⱼ ermittelt.

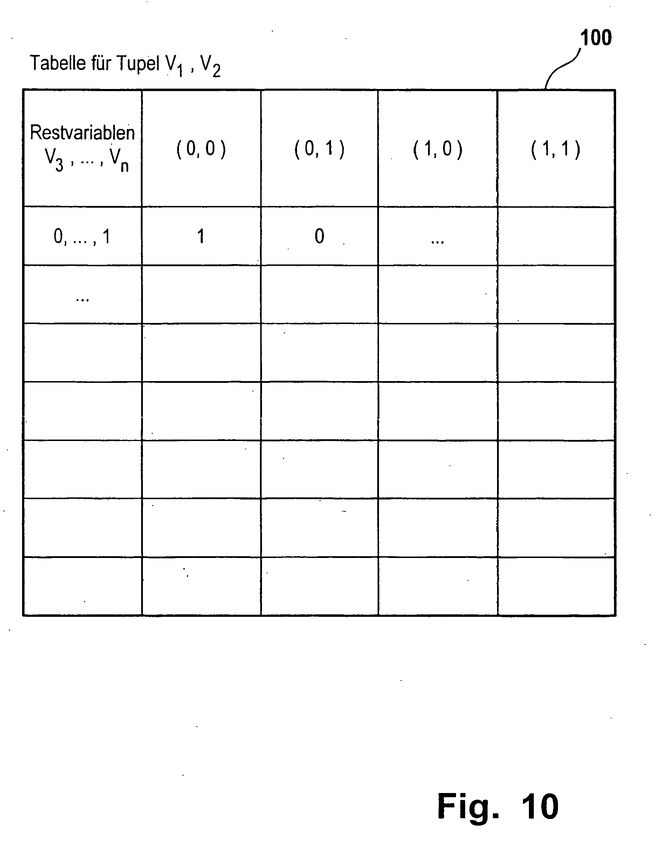

In dem Schritt 91 wird dann für jedes der in dem Schritt 90 ermittelten Zwei-Tupel eine Tabelle gebildet. Die Struktur dieser Tabelle ist in der Figur 10 dargestellt:

Die Figur 10 zeigt eine Tabelle 100, in der als Spaltenindex die möglichen Belegungen der Variablen Vᵢ und Vⱼ dienen. Es gibt also für die zwei Variablen Vᵢ, Vⱼ vier verschiedene Belegungspaare, nämlich (0,0), (0,1), (1,0), (1,1). Das in der Figur 10 gezeigt Beispiel einer solchen Tabelle 100 bezieht sich dabei auf ein Zwei-Tupel von Variablen Vᵢ, Vⱼ mit i = 1 und j = 2.

Als Zeilenindex dienen in der Tabelle 100 die möglichen Belegungen von Restvariablen. Als Restvariablen werden hier sämtliche Variablen bezeichnet mit einem Index, welcher ungleich i und welcher ungleich j ist. In dem betrachteten Beispielsfall der Figur 10 sind dies also die Restvariablen V₃, V₄, ..., Vₙ. Jeder Zeile in der Tabelle 100 ist also eine bestimmte Belegung dieser Restvariablen zugeordnet.

Der Inhalt einer Zelle einer bestimmten Zeile und Spalte der Tabelle 100 ergibt sich dann wie folgt:

Für die Belegung der Restvariablen der betreffenden Zeile und für die Belegung des Zwei-Tupels Vᵢ, Vⱼ der betreffenden Spalte wird auf die Tabelle 80 (vgl. Figur 8) zugegriffen, um für diese Belegung der Variablen V₁, V₂, ..., Vₙ den Wert des Datenfelds Rp zu ermitteln. Dieser Wert des Datenfelds m Rp wird dann in die betreffende Zelle in der Tabelle 100 übernommen.

Nachdem in dem Schritt 91 der Figur 9 für jedes der Zwei-Tupel Vᵢ, Vⱼ eine Tabelle entsprechend der Tabelle 100 der Figur 10 gebildet worden ist, wird in dem Schritt 92 für jede dieser Tabellen die Anzahl der unterschiedlichen Spalten ermittelt.

In dem Schritt 93 wird dann für jede der Tabellen geprüft, ob die Anzahl der unterschiedlichen Spalten einer betrachteten Tabelle 1 beträgt, das heißt, es wird geprüft, ob die einem bestimmten Zwei-Tupel Vᵢ, Vⱼ von Variablen zugeordnete Tabelle aus lauter gleichen Spalten besteht. Wenn dies der Fall ist, so ergibt sich im Schritt 94, dass die betreffenden Variablen Vᵢ, Vⱼ nicht relevant sind.

Im gegenteiligen Fall wird für die betrachtete Tabelle geprüft, ob die Anzahl der unterschiedlichen Spalten zwei beträgt. Wenn dies der Fall ist, so ergibt sich in dem Schritt 96 daraus, dass die betreffenden Variablen Vᵢ und Vⱼ zu einer Wirkeinheit mit genau zwei Eingängen gehören.

Im gegenteiligen Fall werden in dem Schritt 97 die ternären Variationen gebildet. Die Schritte 93 und gegebenenfalls 95 werden für sämtliche der in dem Schritt 91 gebildeten Tabellen durchgeführt, um möglichst bereits an dieser Stelle Variablen als irrelevant zu eliminieren oder Variablen einer Wirkeinheit mit genau zwei Eingängen zuzuordnen. Für die auf diese Art und Weise entweder bereits als irrelevant eliminierten Variablen oder einer Wirkeinheit mit genau zwei Eingängen zugeordneten Variablen erübrigt sich dann die Bestimmung der ternären Variationen des Schritts 97. Im Schritt 97 ist es also lediglich erforderlich, die ternären Variationen für diejenigen Variablen zu bestimmen, die weder in dem Schritt 94 als irrelevant eliminiert werden konnten, noch in dem Schritt 96 einer Wirkeinheit mit genau zwei Eingängen zugeordnet werden konnten.

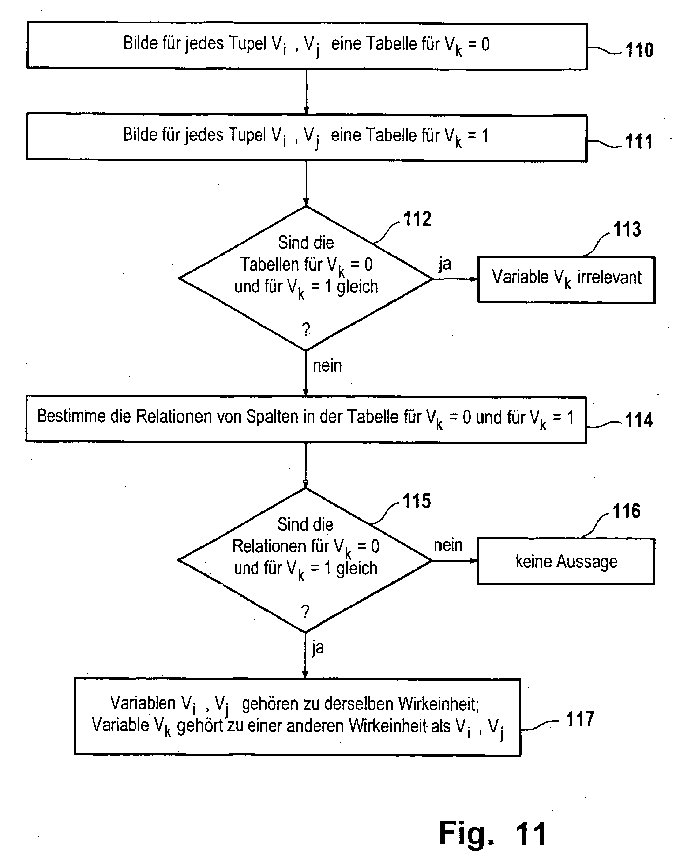

Die Figur 11 zeigt eine Ausführungsform für die Bestimmung der ternären Variationen (vgl. Schritt 97 der Figur 9).

In dem Schritt 110 wird für jedes Zwei-Tupel Vᵢ, Vⱼ eine Tabelle der Form der Tabelle 100 (vgl. Figur 10) gebildet, und zwar für eine Belegung von der Variablen Vₖ mit "Null". Eine solche Tabelle wird also für alle Drei-Tupel Vᵢ, Vⱼ und Vₖ gebildet, wobei Vₖ immer mit Null belegt ist.

In dem Schritt 111 werden entsprechende Tabellen für jedes Tupel Vᵢ, Vⱼ gebildet, und zwar mit einer Belegung von Vₖ = Eins.

In dem Schritt 112 wird geprüft, ob für ein bestimmtes Tupel Vᵢ, Vⱼ, das heißt, für eine bestimmte Wahl von i und j die beiden entsprechenden Tabellen, das heißt, die Tabellen für Vₖ = 0 (Schritt 110) und für Vₖ = 1 (Schritt 111) identisch sind. Wenn dies der Fall ist, folgt daraus in dem Schritt 113, dass die Variable Vₖ als irrelevant eliminiert werden kann.

Wenn das Gegenteil der Fall ist, wird für die beiden betrachteten Tabellen in dem Schritt 114 jeweils die Spaltenrelation bestimmt. Zur Bestimmung einer Spaltenrelation wird so vorgegangen, dass mit Bezug auf eine bestimmte Spalte in einer Tabelle festgestellt wird, in welcher Beziehung die Elemente dieser Spalte zu entsprechenden Elementen derselben Zeile in einer anderen Spalte derselben Tabelle stehen, das heißt, ob diese Elementenpaare in einem Gleichheits- oder Ungleichheitsverhältnis stehen. Diese Gleichheits- bzw. Ungleichheitsverhältnisse werden für jede der Tabellen in dem Schritt 114 mit Bezug auf sämtliche Spalten der betreffenden Tabelle ermittelt.

In dem Schritt 115 wird dann geprüft, ob diese Spaltenrelationen in den Tabellenpaaren für Vₖ=0 und Vₖ=1, die zu demselben Zwei-Tupel Vᵢ, Vⱼ von Variablen gehören, gleich sind. Ist dies nicht der Fall, so ist in dem Schritt 116 keine Aussage möglich. Wenn dies der Fall ist, so folgt daraus in dem Schritt 117, dass es sich bei den Variablen Vᵢ und Vⱼ um einen Variablenpaar-Kandidaten für die Zuordnung zu derselben Wirkeinheit handelt, wobei es sich bei der Wirkeinheit um eine Wirkeinheit mit zwei oder mehr Variablen handeln kann. Ferner folgt daraus in dem Schritt 117, dass - wenn es sich bei den Variablen Vᵢ, Vⱼ um einen zutreffenden Variablenpaar-Kandidaten handelt - die Variable Vₖ zu einer anderen Wirkeinheit als der Wirkeinheit der Variablen Vᵢ und Vⱼ gehören muss.

Als Ergebnis des Verfahrens der Figur 11 resultiert eine Liste von Variablenpaar-Kandidaten Vᵢ und Vⱼ sowie für jeden Variablenpaar-Kandidaten eine Menge von Variablen Vₖ die - falls der betreffende Variablenpaar-Kandidat zutreffend ist - einer anderen Wirkeinheit zugeordnet sein müssen. In der Vereinigungsmenge der Mengen von Variablen Vₖ, die jeweils einen bestimmten Variablenpaar-Kandidaten zugeordnet sind, werden dann widerspruchsfreie Cluster von identischen Mengen von Variablen gesucht. Daraus ergibt sich dann unmittelbar die gesuchte Struktur des Pharmakophors.

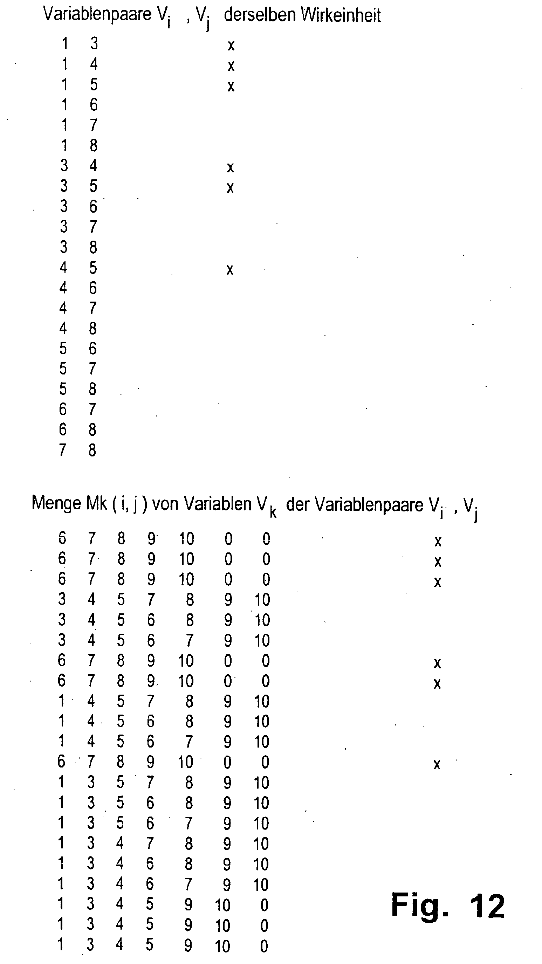

Die Figur 12 zeigt ein entsprechendes Ergebnis, welches durch eine Anwendung des Verfahrens der Figur 11 für einen konkreten Anwendungsfall gewonnen worden ist. In dem konkreten Anwendungsfall wurden aus 1024 Datensätzen 360 relevante ternäre Variationen extrahiert. Jeder Deskriptor des Datensatzes hat eine Anzahl von zehn verschiedenen Variablen (V₁, V₂, ..., V₁₀), die Variable V₂ wurde als irrelevant identifiziert. Die Variablen V₉ und V₁₀ wurden als zu einer Wirkeinheit mit genau zwei Variablen gehörig identifiziert (vgl. Schritt 96 der Figur 9).

Als verbleibende relevante Variablen-Tupel blieben dann nach Elimination der irrelevanten Variablen und der Variablen der Zweier-Wirkeinheit die Variablenpaare Vᵢ und Vⱼ als Kandidaten übrig. Diese sind in der oberen Tabelle der Figur 12 gezeigt.

In der unteren Tabelle der Figur 12 ist in jeder Zeile eine Menge von Variablen Vₖ angegeben, die zu der entsprechenden Zeile der oberen Tabelle der Figur 2, das heißt zu einem bestimmten Variablenpaar-Kandidaten (Vi, Vj), gehört. In der unteren Tabelle der Figur 12 indiziert Null immer eine Lehrstelle. Aus der unteren Tabelle Mk (i, j) wurde die Verteilung der Restvariablen mit

| | | | | |
|---|---|---|---|---|
| Wirkungseinheit 2: | 1 | 3 | 4 | 5 |
| Wirkungseinheit 3: | 6 | 7 | 8 | |

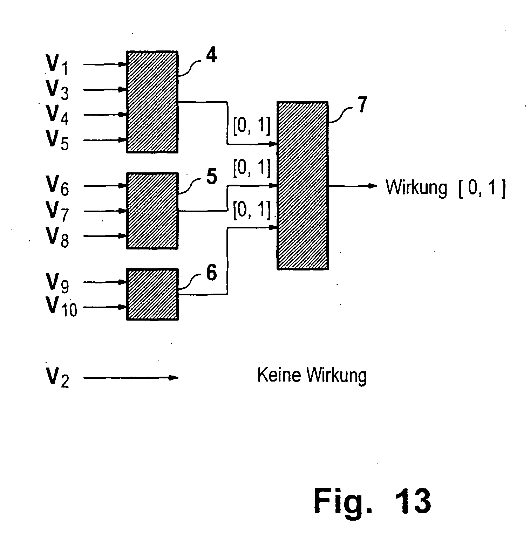

identifiziert. Der entsprechende Cluster ist in den Tabellen der Figur 12 durch ein "x" markiert. Der dem Cluster entsprechende Pharmaphokor mit den Wirkeinheiten 4, 5, 6 und 7 ist in der Figur 13 dargestellt. Die Belegung der Wirkeinheit mit den Variablen V₁, V₃, V₄ und V₅ ergibt sich aus der oberen Tabelle der Figur 12 und die Belegung der Wirkeinheit 5 ergibt sich aus dem für die Menge Mk(i,j) gebildeten Cluster. Die Variablen V₉ und V₁₀ sind der Wirkeinheit mit genau zwei Eingängen zugeordnet und die Variable V₂ ist keiner Wirkeinheit zugeordnet, da diese keine Auswirkung auf die Gesamtwirkung, das heißt, den Ausgang der Wirkeinheit 7, hat.

### Bezugszeichenliste

- Datenbank: 1
- Datenbank: 2
- Pharmakophor: 3
- Wirkungseinheit: 4
- Wirkungseinheit: 5
- Wirkungseinheit: 6
- Wirkungseinheit: 7
- Tabelle: 80
- Tabelle: 100

## Patentansprüche

1. Computerbasiertes Verfahren zur Identifikation eines Pharmakophors mit folgenden Schritten:
- Eingabe von Deskriptoren von Substanzen, wobei jeder Deskriptor durch binäre Variablen (V₁, V₂, ..., Vₙ) beschrieben wird, und Eingabe von den Deskriptoren zugeordneten Wirkungen (Rₚ),
- Bestimmung von binären Variationen für Zwei-Tupel von Variablen (Vᵢ, Vⱼ),
- Bildung einer Tabelle von Wirkungen für jedes der Zwei-Tupel, wobei Permutationen der Restvariablen und die möglichen Belegungen des Zwei-Tupels von Variablen als Tabellenindex verwendet wird,
- Bestimmung der Anzahl der unterschiedlichen Spalten für jede einem Zwei-Tupel zugeordnete Tabelle,
- Zuordnung von einem Zwei-Tupel von Variablen als Variablenpaar zu der Wirkeinheit, die genau zwei Variablen aufweist wenn die Anzahl der unterschiedlichen Spalten der entsprechenden Tabelle zwei beträgt.
- Bestimmung von ternären Variationen zu Drei-Tupel von Variablen (Vᵢ, Vⱼ, Vₖ),
- Bestimmung von Variablenpaar-Kandidaten aus den ternären Variationen zur Zuordnung zu einer gemeinsamen Wirkeinheit, wobei die gemeinsame Wirkeinheit zwei oder mehr Variablen aufweist, und Bestimmung einer Menge von Variablen für jeden Variablenpaar-Kandidaten, die solche Variablen beinhaltet, die bei der Zuordnung des Variablenpaar-Kandidaten zu der gemeinsamen Wirkeinheit einer von der gemeinsamen Wirkeinheit verschiedenen Wirkeinheit zugeordnet sein müssen,
- Bestimmung eines konfliktfreien Clusters der Mengen von Variablen zur Identifizierung der gemeinsamen Wirkeinheit.

2. Verfahren nach Anspruch 1, wobei es sich bei den Deskriptoren um binäre Deskriptoren einer Substanzbibliothek handelt.

3. Verfahren nach Anspruch 1 oder 2 mit einem Schritt zur Datenkompression der binären Deskriptoren.

4. Verfahren nach Anspruch 1, 2 oder 3, wobei es sich bei den Wirkungen um die Wirkungen der den Deskriptoren jeweils zugeordneten Substanzen auf ein Zielmolekül handelt, und die Wirkungen binär codiert sind.

5. Verfahren nach einem der Ansprüche 1 bis 4, wobei die Variablen eines Zwei-Tupels, für das die Anzahl der unterschiedlichen Spalten der entsprechenden Tabelle eins beträgt, als irrelevant eliminiert werden.

6. Verfahren nach einem der Ansprüche 1 bis 5, wobei die ternären Variationen nur dann bestimmt werden, wenn es Tabellen gibt, für die die Anzahl der unterschiedlichen Spalten drei und mehr beträgt.

7. Verfahren nach einem der vorhergehenden Ansprüche 1 bis 6, wobei zur Bestimmung der ternären Variationen und zur Bestimmung von Variablenpaar-Kandidaten zur Zuordnung zu einer gemeinsamen Wirkeinheit die folgenden Schritte durchgeführt werden:
- Bildung von ersten Tabellen für Zwei-Tupel von Variablen (Vᵢ, Vⱼ) und für eine erste Wirkung einer weiteren Variablen (Vₖ),
- Bildung von zweiten Tabellen für Zwei-Tupel von Variablen (Vᵢ, Vⱼ), und für eine zweite Wirkung einer weiteren Variablen (Vₖ),
- Bestimmung von Spaltenrelationen einander entsprechender erster und zweiter Tabellen mit unterschiedlichen Wirkungen der weiteren Variablen,
- Ermittlung von Variabelnpaar-Kandidaten und der Menge von Variablen aus den entsprechenden ersten und zweiten Tabellen, die gleiche Spaltenrelationen aufweisen.

8. Verfahren nach Anspruch 7, wobei eine weitere Variable als irrelevant eliminiert wird, wenn die ersten und zweiten Tabellen dieser weiteren Variablen im Wesentlichen gleich sind.

9. Verfahren nach Anspruch 7 oder 8, wobei in einem konfliktfreiem Cluster die Menge von Variablen der konfliktfreien Variablenpaar-Kandidaten identisch sind.

10. Verfahren nach einem der vorhergehenden Ansprüche 1 bis 9, wobei zur Eliminierung irrelevanter Variablen, zur Bildung von binären Variationen und/oder zur Bildung von ternären Variationen Toleranzen zugelassen werden.

11. Verfahren nach einem der vorhergehenden Ansprüche 1 bis 10, wobei automatisch Zulässigkeitsschranken, die konfliktfreie Lösungen ergeben, ausgewählt werden, basierend auf einem Absuchen des dreidimensionalen Parameterraums.

12. Computerprogrammprodukt mit Programmmitteln zur Durchführung eines Verfahrens nach einem der vorhergehenden Ansprüche 1 bis 11.

13. Computersystem mit Mitteln zur Durchführung eines Verfahrens nach einem der vorhergehenden Ansprüche 1 bis 11.

## Claims

1. Computer-based method for identifying a pharmacophore having the following steps:
- inputting of descriptors of substances, each descriptor being described by binary variables (V₁, V₂, ..., Vₙ), and inputting of effects (Rₚ) assigned to the descriptors,
- determination of binary variations for two-tuples of variables (Vᵢ, Vⱼ),
- formation of a table of effects for each of the two-tuples, permutations of the remaining variables and the possible allocations of the two-tuples of variables being used as a table index,
- determination of the number of different columns for each table which is assigned to a two-tuple,
- assignment of a two-tuple of variables as a pair of variables to the active entity which has precisely two variables if the number of different columns of the corresponding table is two,
- determination of ternary variations to three-tuples of variables (Vᵢ, Vⱼ, Vₖ),
- determination of variable pair candidates from the ternary variations for assignment to a common active entity, the common active entity having two or more variables, and determination of a set of variables for each variable pair candidate which contains such variables which, when the variable pair candidate is assigned to the common active entity, have to be assigned to an active entity other than the common active entity,
- determination of a conflict-free cluster of the sets of variables for identification of the common active entity.

2. Method according to Claim 1, the descriptors being binary descriptors of a substance library.

3. Method according to Claim 1 or 2, having a step for performing data compression on the binary descriptors.

4. Method according to Claim 1, 2 or 3, the effects being the effects of the substances which are respectively assigned to the descriptors on a target molecule, and the effects being binary coded.

5. Method according to one of Claims 1 to 4, the variables of a two-tuple for which the number of different columns of the corresponding table is one being eliminated as irrelevant.

6. Method according to one of Claims 1 to 5, the ternary variations being determined only if there are tables for which the number of different columns is three and more.

7. Method according to one of the preceding Claims 1 to 6, in which, in order to determine the ternary variations and to determine variable pair candidates for assignment to a common active entity, the following steps are carried out:
- formation of first tables for two-tuples of variables (Vᵢ, Vⱼ) and for a first effect of a further variable (Vₖ),
- formation of second tables for two-tuples of variables (Vᵢ, Vⱼ), and for a second effect of a further variable (Vₖ),
- determination of column relations of corresponding first and second tables with different effects of the further variable,
- determination of variable pair candidates and of the set of variables from the corresponding first and second tables which have identical column relations.

8. Method according to Claim 7, a further variable being eliminated as irrelevant if the first and second tables of this further variable are essentially the same.

9. Method according to Claim 7 or 8, the set of variables of the conflict-free variable pair candidates being identical in a conflict-free cluster.

10. Method according to one of the preceding Claims 1 to 9, tolerances being permitted in order to eliminate irrelevant variables, to form binary variations and/or to form ternary variations.

11. Method according to one of the preceding claims 1 to 10, automatic permissibility limits which yield conflict-free solutions being selected on the basis of searching the three-dimensional parameter space.

12. Computer program product having programming means for carrying out a method according to one of the preceding Claims 1 to 11.

13. Computer system having means for carrying out a method according to one of the preceding Claims 1 to 11.

## Revendications

1. Méthode informatique d'identification d'un pharmacophore comportant les étapes suivantes:
- Introduction de descripteurs de substances, chaque descripteur étant décrit par des variables binaires (V₁, V₂, ..., Vₙ), et introduction d'effets affectés aux descripteurs (Rₖ),
- détermination de variations binaires pour des couples de variables, (Vi, Vj),
- formation d'un tableau d'effets pour chacun des couples, des permutations des variables restantes et les affectations possibles du couple de variables étant utilisées comme index de tableau,
- détermination du nombre de colonnes différentes pour chaque tableau correspondant à un couple,
- affectation d'un couple de variables comme paire de variables à l'unité d'action, qui présente exactement deux variables, si le nombre de colonnes différentes du tableau correspondant est de deux.
- détermination de variations ternaires pour des triplets de variables (Vᵢ, Vⱼ, Vₖ),
- détermination de paires de variables candidates des variations ternaires pour l'affectation à une unité active commune, l'unité active commune présentant deux variables ou plus, et détermination d'une quantité de variables pour chaque paire de variables candidate - candidates qui contiennent des variables qui, lors de l'affectation de la paire de variables candidate à l'unité active commune, doivent être affectées à une unité active différente de l'unité active commune,
- détermination d'un groupe sans conflit des quantités de variables pour l'identification de l'unité active commune.

2. Méthode suivant la revendication 1, les descripteurs étant des descripteurs binaires d'une bibliothèque de substances.

3. Méthode suivant la revendication 1 ou 2 avec une étape de compression de données des descripteurs binaires.

4. Méthode suivant la revendication 1, 2 ou 3, les actions étant les effets des substances sur une molécule visée qui correspondent respectivement aux descripteurs, et les actions étant codées sous forme binaire.

5. Méthode suivant l'une des revendications 1 à 4, dans laquelle les variables d'un couple pour lequel le nombre de colonnes différentes du tableau correspondant est de un sont éliminées comme non pertinentes.

6. Méthode suivant l'une des revendications 1 à 5, les variations ternaires étant uniquement déterminées s'il existe des tableaux pour lesquels le nombre de colonnes différentes est de trois ou plus.

7. Méthode suivant l'une des revendications précédentes 1 à 6, les étapes suivantes étant exécutées pour la détermination des variations ternaires et pour la détermination de paires de variables candidates pour l'affectation à une unité active commune:
- Formation de premiers tableaux pour des couples de variables (Vᵢ, Vⱼ) et pour une première action d'une variable supplémentaire (Vₖ),
- Formation de deuxièmes tableaux pour des couples de variables (Vᵢ, Vⱼ) et pour une deuxième action d'une variable supplémentaire (Vₖ),
- Détermination de relations entre colonnes de premiers et deuxièmes tableaux se correspondant avec des actions différentes des variables supplémentaires,
- Détermination de paires de variables candidates et de la quantité de variables des premiers et deuxièmes tableaux correspondant qui présentent les mêmes relations entre colonnes.

8. Méthode suivant la revendication 7, une variable supplémentaire étant éliminée comme non pertinente si les premiers et deuxièmes tableaux de cette variable supplémentaire sont essentiellement identiques.

9. Méthode suivant la revendication 7 ou 8, la quantité de variables des paires de variables candidates étant identique dans un groupe sans conflit.

10. Méthode suivant l'une des revendications précédentes 1 à 9, des tolérances étant admises pour l'élimination de variables non pertinentes, pour la formation de variations binaires et/ou la formation de variations ternaires.

11. Méthode suivant l'une des revendications précédentes 1 à 10, des barrières d'admission, qui donnent des solutions sans conflit, étant automatiquement sélectionnées, sur base d'une recherche dans l'espace tridimensionnel des paramètres.

12. Programme d'ordinateur avec des moyens de programme pour la réalisation d'une méthode suivant l'une des revendications précédentes 1 à 11.

13. Système informatique avec des moyens de réalisation d'une méthode suivant l'une des revendications précédentes 1 à 11.
